# FASCICULE DE BREVET EUROPEEN

(11) **EP 1 513 583 B1**
(45) Date de publication et mention de la délivrance du brevet: **23.06.2010**
(21) Numéro de dépôt: 03775479.3
(22) Date de dépôt: 09.10.2003
(51) Int. Cl.: A61M 39/10, F16L 37/084

(54) **CONNECTEUR DE FLUIDE A USAGE MEDICAL ET SES APPLICATIONS**
MEDIZINISCHE SCHLAUCHKUPPLUNG FÜR FLÜSSIGKEITEN UND DEREN ANDWENDUNGEN
FLUID CONNECTOR FOR MEDICAL USE AND USES THEREOF

(30) Priorité: 10.10.2002 FR 0212581
(43) Date de publication de la demande: 16.03.2005
(73) Titulaire: VYGON, 95440 Ecouen (FR)
(72) Inventeur: DALLE, Valéry, F-60270 Gouvieux (FR); GUYMARC'H, Pierrick, F-95120 Ermont (FR); CARREZ, Jean-Luc, F-95440 Ecouen (FR)
(74) Mandataire: Texier, Christian
(86) Numéro de dépôt international: PCT/FR2003/002979
(87) Numéro de publication internationale: WO 2004/033023

(56) Documents cités:
- EP-A- 0 633 038
- EP-A- 0 998 956
- EP-A- 1 070 899
- FR-A- 2 780 765

## Description

L'invention concerne un connecteur pour circuit de fluide à usage médical.

Elle s'applique en particulier aux assemblages coniques des seringues, aiguilles et autres appareils à usage médical, par exemple les équipements de transfusion. FR-2 780 765 divulgue un connecteur, à embases non coniques, avec une colerette de verrouillage (fig. 6 et 7) qui ne possède pas de lumières, mais des ergots 48.

Ces assemblages utilisent généralement des raccords comportant une embase mâle et une embase femelle qui s'emboîtent par des extrémités coniques (dites cônes luer) avec étanchéité latérale autour des extrémités emboîtées.

La sécurité du raccordement est parfois renforcée par un vissage.

En pratique, on constate deux inconvénients :
- les raccords peuvent se dévisser de façon intempestive sous l'effet d'une sollicitation à la rotation de l'une des embases ;
- le même type de raccord étant utilisé pour des applications différentes, il y a de réels risques de confusion pour le personnel soignant, comme monter par exemple une ligne de nutrition dans l'embase femelle d'un cathéter veineux.

Il y a des demandes actuelles pour de nouveaux systèmes à créer évitant ces confusions et sécurisant de plus la connexion pour la rendre indéconnectable sauf par une action volontaire.

Un but de l'invention est de fournir un connecteur permettant de raccorder avec un verrouillage automatique du raccordement.

Un autre but de l'invention est de pouvoir spécialiser le raccord en utilisant des cônes de pentes et de diamètres différents selon les applications, évitant ainsi tout risque de confusion du fait qu'en cas d'erreur, ni la tenue, ni le verrouillage ne sont possibles.

On y parvient, selon l'invention, avec un connecteur de fluide à verrouillage automatique constitué d'une embase mâle tubulaire qui présente une extrémité de raccordement à un embout et une extrémité à emboîter de forme extérieure conique et d'une embase femelle tubulaire qui présente une extrémité de raccordement à un embout et une extrémité de réception de forme intérieure conique apte à recevoir avec étanchéité latérale ladite extrémité à emboîter du raccord mâle, l'embase mâle étant munie d'ergots latéraux extérieurs, l'embase femelle étant munie d'un ensemble de crans extérieurs et le connecteur comprenant une collerette de verrouillage à monter sur les deux embases, ladite collerette présentant des lumières latérales aptes à être traversées par les ergots de l'embase mâle pour le blocage axial et en rotation de la collerette sur l'embase mâle, et ladite collerette présentant une partie déformable transversalement et munie de crans intérieurs, les crans étant conformés pour que les crans de la collerette puissent passer sur les crans de l'embase femelle lorsque la collerette est poussée dans un sens sur l'embase et soient retenus par les crans de l'embase femelle lors d'une traction en sens inverse, et les crans étant conformés pour permettre une rotation relative de l'embase femelle et de la collerette, et les crans de la collerette étant écartables latéralement des crans de l'embase femelle par déformation transversale de la partie de la collerette qui porte les crans.

Le mot « embout » désigne toute extrémité de raccordement d'un appareillage médical.

Il peut s'agir notamment :
- de l'extrémité luer normalisée mâle ou femelle d'un appareil tel qu'une seringue (S), une embase (E) d'aiguille ou de cathéter, etc. ;
- de l'extrémité cylindrique d'un tube (T).

Dans des modes de réalisation préférés, le connecteur présente encore une ou plusieurs des caractéristiques suivantes :
- La collerette comporte deux séries de crans diamétralement opposées et deux zones à 90° des crans aptes à être poussées radialement pour déformer transversalement la collerette afin d'éloigner les deux séries de crans l'une de l'autre.
- Les crans présentent une pente douce pour faciliter le verrouillage par poussée axiale et une pente raide pour empêcher le déverrouillage par traction axiale, le verrouillage étant assuré par le contact de pentes raides des crans de la collerette sur des pentes raides des crans de l'embase femelle.
- Les crans de l'embase femelle sont des cannelures ou portions de cannelures circulaires.

On décrira ci-après des exemples de connecteurs conformes à l'invention, en référence aux figures du dessin joint sur lequel :
- la figure 1 est une vue extérieure de l'embase mâle ;
- la figure 2 est une vue en coupe de l'embase mâle ;
- la figure 3 est une vue extérieure de l'embase femelle ;
- la figure 4 est une vue en coupe de l'embase femelle ;
- la figure 5 est une vue en perspective de la collerette de verrouillage ;
- la figure 6 est une autre vue en perspective de la collerette ;
- les figures 7 et 8 sont des vues en coupe transversale de la collerette, respectivement au repos et déformée ;
- la figure 9 est une vue extérieure du connecteur à l'état verrouillé ;
- la figure 10 est une vue en coupe du connecteur à l'état verrouillé ;
- la figure 11 est une coupe agrandie de la zone de verrouillage du connecteur à l'état verrouillé, et
- les figures 12 à 17 illustrent une application d'un connecteur selon l'invention au raccordement d'une seringue à l'embase d'une aiguille.

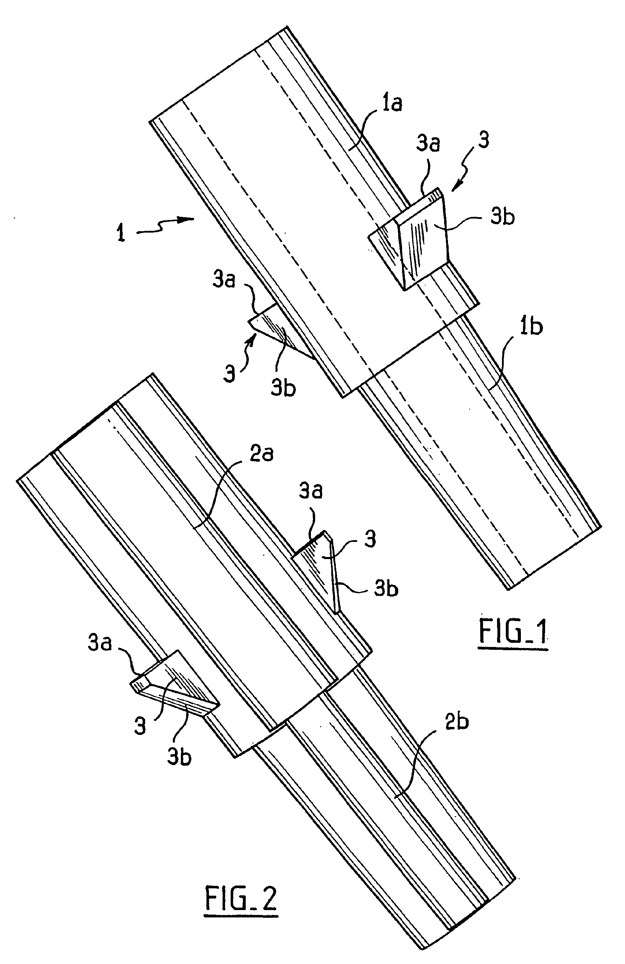

Le dispositif représente sur les figures comporte (figures 1 et 2) une embase tubulaire mâle (1) qui présente une extrémité de raccordement adaptable (1a) et une extrémité opposée à emboîter (1b) (figure 1).

L'embase mâle est traversée par une lumière cylindrique longitudinale (2) qui comprend une partie de lumière (2a) dans l'extrémité d'adaptation et une partie de lumière (2b) dans l'extrémité à emboîter. Le diamètre de la lumière (2a) est plus grand que le diamètre de la lumière (2b).

L'extrémité d'adaptation (1a) porte deux ergots latéraux (3) conformés en triangle situés dans une même section sur deux côtés et diamétralement opposés (figure 1). Chaque ergot présente une pente raide (3a) tournée vers l'extrémité d'adaptation et une pente douce (3b) tournée vers l'extrémité à emboîter.

L'extrémité à emboîter (1b) est conique extérieurement.

L'extrémité d'adaptation est cylindrique extérieurement.

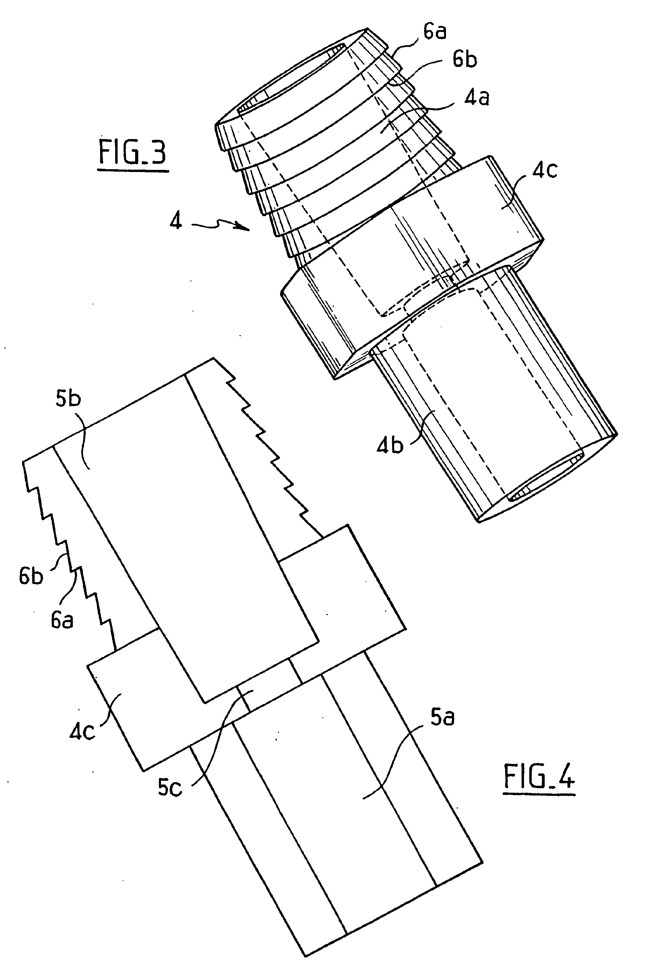

Le dispositif représenté comprend également (figures 3 et 4) une embase tubulaire femelle (4) qui comprend une extrémité de raccordement adaptable (4a) et une extrémité opposée (4b) réceptrice d'emboîtement (figure 3).

L'embase femelle est traversée par une lumière longitudinale (5) qui comprend une partie de lumière (5a) dans l'extrémité d'adaptation (4a) et une partie de lumière (5b) dans l'extrémité réceptrice d'emboîtement (figure 4). La partie de lumière (5a) est moins large que la partie de lumière (5b) et ces deux parties de lumière sont séparées par une partie lumière (5c) cylindrique et plus étroite que deux autres parties de lumière.

L'embase femelle présente un renforcement périphérique (4c) intermédiaire entre ses extrémités (4a) et (4b) pour faciliter sa préhension.

L'extrémité réceptrice d'emboîtement (4b) de l'embase femelle comprend des crans extérieurs (6) constitués dans cet exemple par des cannelures parallèles qui présentent un front incliné (6a) et un front raide (6b). Le front raide est tourné vers l'extrémité d'adaptation (5a).

L'extrémité d'adaptation est cylindrique extérieurement.

Le dispositif comprend également une collerette (7).

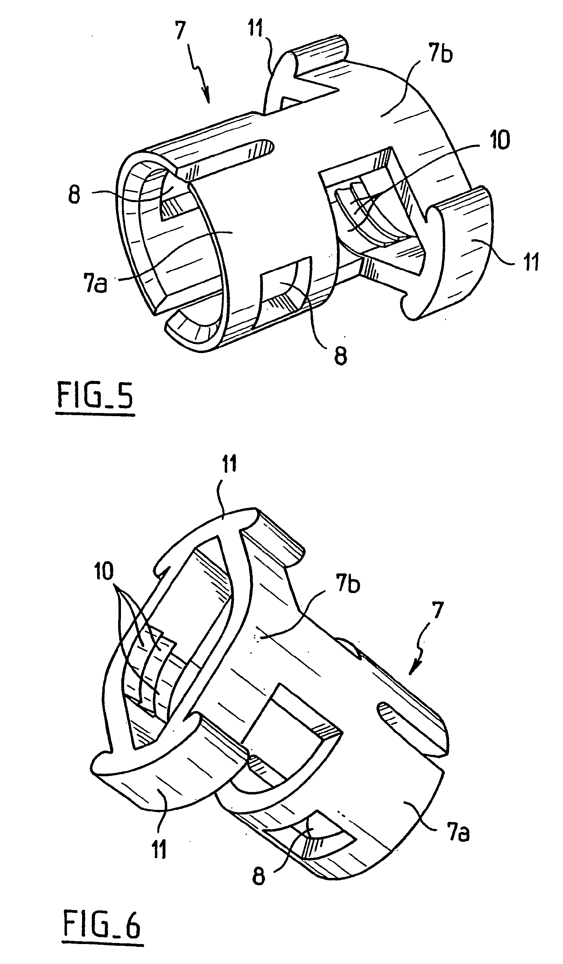

La collerette (7) est un corps tubulaire qui présente (figures 5 à 8) une extrémité (7a) dont la paroi latérale est percée de deux fenêtres (8) aptes à être traversées par les ergots (3) de l'embase mâle lorsque la collerette est enfilée sur cette embase et poussée vers l'embase femelle, et qui présente une extrémité opposée (7b) dont la paroi latérale est munie sur sa face interne (9) de deux séries de crans (10) en vis à vis aptes à coopérer avec les crans de l'embase femelle lorsque la collerette est poussée sur l'embase femelle.

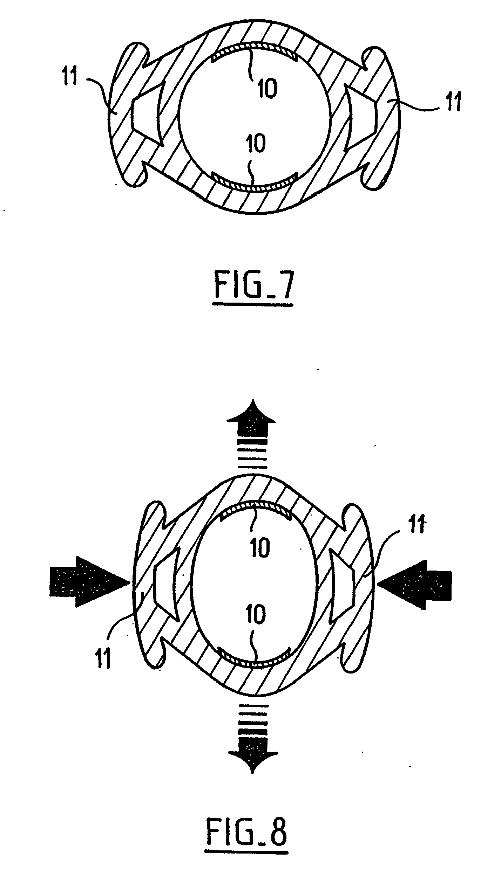

En outre, la section de la collerette qui comporte les crans (10) est élargie vers l'extérieur pour former deux zones (11) d'appui tactile à 90° des crans en sorte qu'un appui radial sur ces zones provoque une déformation de la collerette qui éloigne les crans l'un de l'autre (figure 8). La matière de la collerette est choisie pour permettre cette déformation.

Lors de l'emmanchement du cône mâle dans le cône femelle, les crans de la collerette passent automatiquement par dessus ceux de l'embase femelle, ceci jusqu'à emmanchement complet des deux cônes.

Tout retour en arrière (déconnexion) est rendu impossible par l'engrènement de ces crans. Une rotation en sens inverse des embases femelles et mâles est possible, sans risque de déconnexion.

L'étanchéité est assurée par le contact cône/cône, le verrouillage est assuré par le contact des pentes raides des crans de la collerette sur celles des crans de l'embase femelle.

La pression sur les zones d'appui soulève les crans de la collerette et élimine le contact des pentes raides des crans. En maintenant cet appui, la déconnexion axiale est possible.

Le fait d'avoir à exercer cet effort d'appui pour déconnecter constitue la sécurisation de cette connexion.

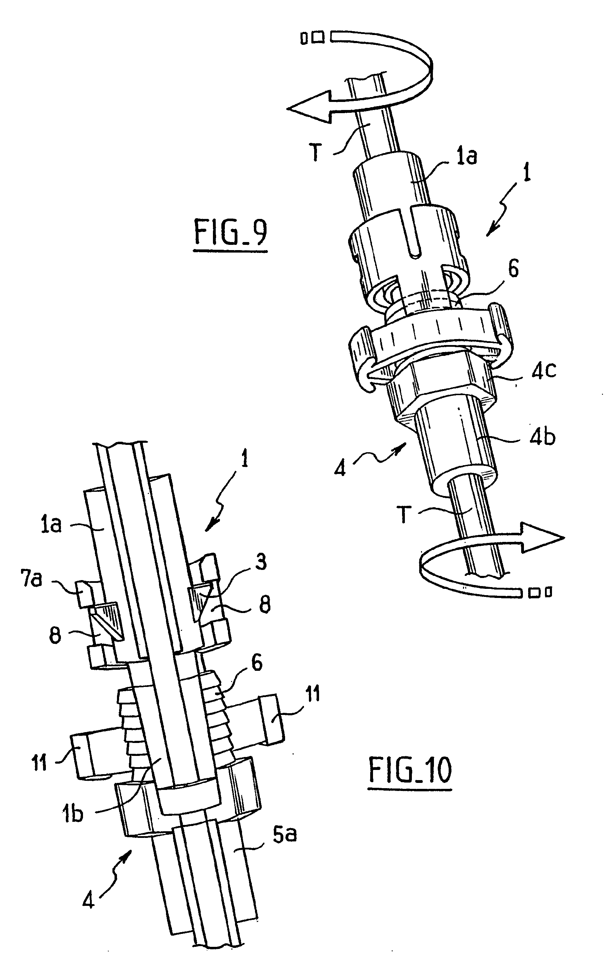

Les figures 8 à 11 montrent la connexion réalisée.

L'invention permet d'adapter le connecteur aux différentes utilisations possibles dans le domaine médical sans risque de confusion, en jouant sur le diamètre intérieur et la forme des extrémités d'adaptation des embases.

L'invention n'est pas limitée aux réalisations décrites.

Cette connexion médicale peut fonctionner avec des cônes mâles et femelles de formes différentes, assurant l'étanchéité, mais aussi la non-montabilité et le non verrouillage entre formes différentes, ceci pour permettre d'éviter le montage entre eux d'équipements, par exemple : accès veineux (artériel, nutrition, péridurale,...), chaque équipement dans un domàine étant équipé du bon cône (mâle ou femelle).

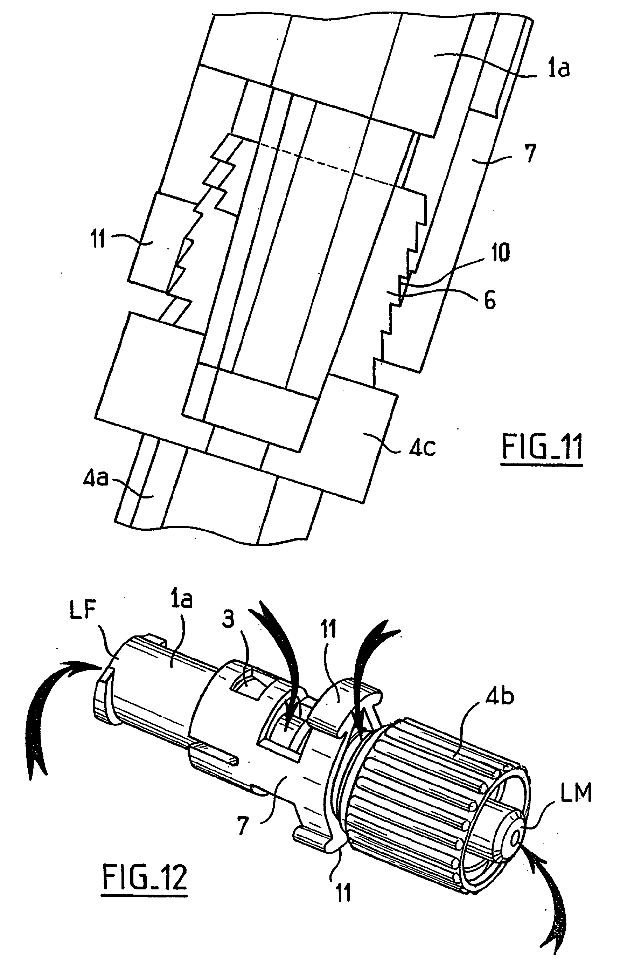

Les figures 12 à 17 illustrent un exemple d'application d'un connecteur à verrouillage selon l'invention pour le raccordement sécurisé d'une seringue S à l'embase E d'une aiguille :
- la figure 12 est une perspective du connecteur dont l'embase mâle a son extrémité d'adaptation (1a) conçue intérieurement avec un cône luer lock femelle LF pour montage (et collage si nécessaire) sur un cône luer mâle d'un appareil à raccorder, en l'espèce une seringue, l'embase femelle du connecteur ayant son extrémité d'adaptation (4b) conçue intérieurement avec un cône luer lock mâle LM permettant son montage (avec un collage si nécessaire) sur un cône luer femelle d'un appareil à raccorder, en l'espèce une aiguille.

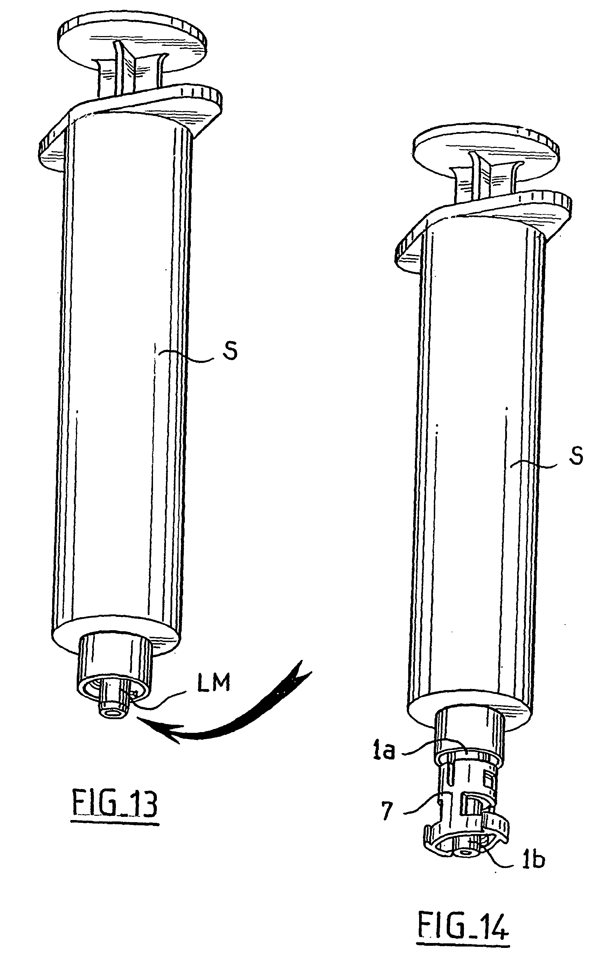

- la figure 13 est une perspective de la seringue conçue à son extrémité de sortie avec un luer mâle LM ;
- la figure 14 est une perspective de l'embase mâle du connecteur monté sur la seringue par emmanchement du luer mâle LM de la seringue S dans l'extrémité d'adaptation (1a) de l'embase mâle, laquelle est constitué intérieurement comme un luer femelle LF ;

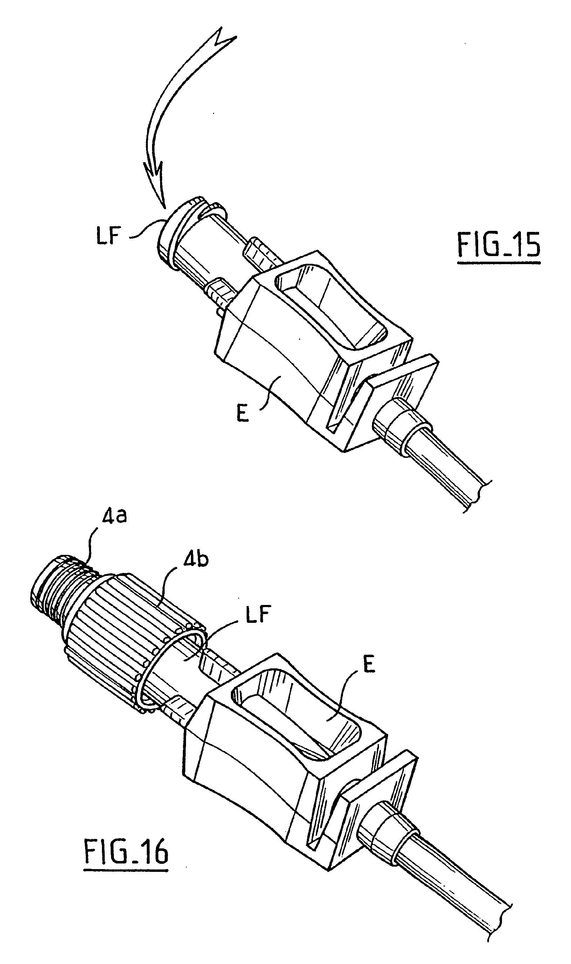

- la figure 15 est une perspective de l'embase E de l'aiguille conçue à son extrémité d'entrée avec un luer femelle LF ;
- la figure 16 est une perspective de l'embase femelle du connecteur montée sur l'embase E de l'aiguille. A cette fin, l'extrémité d'adaptation (4b) de l'embase femelle est constituée intérieurement comme un luer mâle LM apte à être emmanché dans le luer femelle LF de l'embase E de l'aiguille.

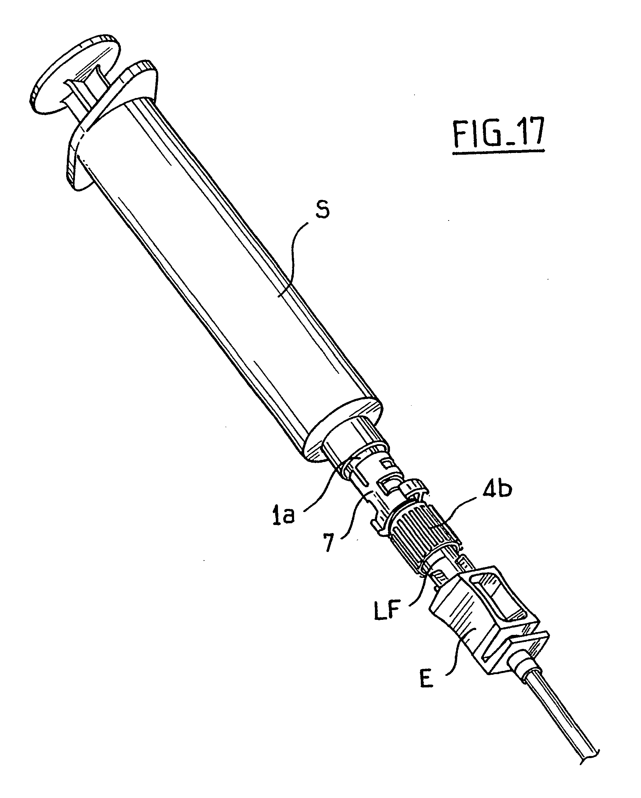

- La figure 17 est une perspective de la seringue raccordée à l'embase de l'aiguille par la connexion de l'invention.

L'invention n'est pas limitée à cet exemple de raccordement.

Ainsi, l'extrémité d'adaptation de l'une des embases d'un connecteur sécurisé selon l'invention peut être raccordée à un tube par emboîtement intérieur ou extérieur, ce tube servant par exemple à établir une liaison avec un appareil.

Il est également prévu par l'invention de fournir une nouvelle génération d'appareils médicaux, tels que notamment tubes, seringues et embases d'aiguille ou de cathéter, présentant une extrémité de raccordement constituée par l'une des embases mâle et femelle du connecteur de l'invention.

Il est enfin prévu par l'invention de remplacer les moyens décrits par des moyens fonctionnellement équivalents ou de réaliser des inversions cinématiques, par exemple en échangeant des moyens entre l'une et l'autre des embases du connecteur.

## Revendications

1. Connecteur de fluide à verrouillage automatique pour raccorder les cônes luer de deux appareils dans le domaine médical, constitué d'une embase mâle tubulaire (1) qui présente une extrémité d'adaptation (1a) à un embout et une extrémité à emboîter (1b) de forme extérieure conique et d'une embase femelle tubulaire (4) qui présente une extrémité d'adaptation (4a) à un embout et une extrémité de réception (4b) de forme intérieure conique apte à recevoir avec étanchéité latérale ladite extrémité à emboîter du raccord mâle, et le connecteur comprenant une collerette de verrouillage (7) à monter sur les deux embases, ladite collerette présentant des lumières latérales (8) aptes à être traversées par des ergots latéraux extérieurs (3) de l'embase mâle pour le blocage axial et en rotation de la collerette sur l'embase mâle, et présentant une partie (76) déformable transversalement munie de crans intérieurs (10), conformés pour que des crans intérieurs (10) de la collerette puissent passer sur les crans (6) de l'embase femelle lorsque la collerette est poussée dans un sens sur l'embase et soient retenus par les crans (6) de l'embase femelle lors d'une traction en sens inverse, et les crans de la collerette et de l'embase femelle étant conformés pour permettre une rotation relative de l'embase femelle et de la collerette, lesdits crans (10) de la collerette étant écartables latéralement des crans (6) de l'embase femelle par déformation transversale de la partie (7b) de la collerette qui porte les crans.

2. Connecteur selon 1a revendication 1 dont ladite collerette comporte deux séries de crans (10) diamétralement opposées et deux zones (11) à 90° des crans aptes à être poussées radialement pour déformer transversalement la collerette afin d'éloigner les deux séries de crans l'une de l'autre pour permettre la séparation de la collerette et de l'embase femelle du connecteur.

3. Connecteur selon la revendication 1 ou 2 dont les crans (6, 10)de la collerette et de l'embase femelle présentent une pente douce (6a, 10a) pour faciliter et automatiser le verrouillage par poussée axiale et une pente raide (6b, 10b) pour empêcher le déverrouillage, le verrouillage étant assuré par le contact des pentes raides des crans de la collerette sur les pentes raides des crans de l'embase femelle.

4. Connecteur selon l'une des revendications 1 à 3 dans lequel les crans (6) de l'embase femelle sont des cannelures ou des portions de cannelures circulaires.

5. Connecteur selon l'une des revendications 1 à 4 dont l'embase mâle (1) a son extrémité d'adaptation (1a) conçue pour recevoir à emmanchement un luer mâle (LM).

6. Connecteur selon l'une des revendications 1 à 4 dont l'embase femelle (4) à son extrémité d'adaptation (4a) conçue intérieurement avec un luer mâle (LM) apte à être emmanché dans un luer femelle (LF).

7. Connecteur selon l'une des revendications 1 à 4 dont l'une et/ou l'autre des extrémités d'adaptation sont conçues pour être raccordées à un tube cylindrique.

8. Application d'un connecteur selon l'une des revendications 1 à 7 au raccordement d'une seringue ou d'une embase d'aiguille ou de cathéter.

9. Application d'un connecteur selon l'une des revendications 1 à 7 au raccordement d'un tube.

## Claims

1. Connector for fluids with automatic locking for connecting the Luer cones of two pieces of equipment for medical use, constituted of a tubular male socket (1) having an adaptation tip (1a) at one end-piece and a tip to be fitted (1b) of external conical shape, and a tubular female socket having an adaptation tip (4a) at one end-piece and a reception tip (4b) of internal conical shape able to receive said fitting tip of the male connection with lateral seal, and the connector comprising a locking collar (7) to be mounted on the two sockets, said collar having lateral ports (8) able to be crossed by external lateral lugs (3) of the male socket for blocking the collar axially and in rotation on the male socket, and having a transversally deformable part (7b) equipped with internal notches (10), shaped so that the internal notches (10) of the collar can pass over the notches (6) of the female socket when the collar is pushed in a direction towards the socket and be held by the notches (6) of the female socket when there is traction in the reverse direction, and the notches of the collar and of the female socket being shaped so as to allow relative rotation of the female socket and the collar, said notches (6) of the collar being able to be separated laterally from the notches (6) of the female socket by transversal deformation of the part (7b) of the collar carrying the notches.

2. Connector according to claim 1 wherein said collar comprises two sets of notches (10) diametrically opposite each other and two zones (11) at 90° relative to the notches able to be pushed radially to deform the collar transversally in order to separate the two series of notches from each other in order to allow separation of the collar and the female socket from the connector.

3. Connector according to either one of claims 1 and 2 wherein the notches (6, 10) of the collar and the female socket have a slight inclination (6a, 10a) to facilitate and automate the locking by axial thrust and a steep slope (6b, 10b) to prevent unlocking, the locking being ensured by the contact between the steep inclinations of the notches of the collar on the steep inclinations of the notches of the female socket.

4. Connector according to any one of claims 1 to 3 wherein the notches (6) of the female socket are grooves or portions of circular grooves.

5. Connector according to any one of claims 1 to 4 wherein the male socket (1) has its adaptation tip (1a) designed to receive a male Luer (LM) by fitting.

6. Connector according to any one of claims 1 to 4 wherein the female socket (4) has its adaptation tip (4a) designed internally with a male Luer (LM) able to be fit into a female Luer (LF).

7. Connector according to any one of claims 1 to 4 wherein one and/or the other of the adaptation tips are designed to be connected to a cylindrical tube.

8. Application of a connector according to any one of claims 1 to 7 for connection of a syringe or a needle or catheter hub.

9. Application of a connector according to any one of claims 1 to 7 for connection of a tube.

## Patentansprüche

1. Verbinder für Flüssigkeiten mit automatischer Verriegelung zur Verbindung von Luer-Lock-Konen von zwei Vorrichtungen im medizinischen Bereich, wobei der Verbinder aus einem rohrförmigen Steckerteil (1), das ein Ende zum Einpassen (1a) an ein Ansatzstück und ein Einfügungsende (1b) mit konischer Außenform aufweist, und einem rohrförmigen Buchsenteil (4) besteht, das ein Ende zum Einpassen (4a) an ein Ansatzstück und ein Aufnahmeende (4b) mit konischer Innenform aufweist, das dazu geeignet ist, das genannte Einfügungsende der Steckverbindung mit seitlicher Dichtigkeit aufzunehmen, und der Verbinder einen Verriegelungskragen (7) zur Montage auf den zwei Teilen umfasst, wobei der genannte Kragen seitliche Öffnungen (8) aufweist, die dazu geeignet sind, von seitlichen Außenstiften (3) des Steckerteils zur axialen und Drehblockade des Kragens auf dem Steckerteil durchquert zu werden, und einen quer verformbaren Abschnitt (7b) aufweist, der mit Innenkerben (10) versehen ist, die so gestaltet sind, dass die Innenkerben (10) des Kragens über die Kerben (6) des Buchsenteils hinweggehen können, wenn der Kragen in einer Richtung auf dem Teil geschoben wird, und bei einem Zug in umgekehrter Richtung von den Kerben (6) des Buchsenteils gehalten werden, und die Kerben des Kragens und des Buchsenteils zum Ermöglichen einer relativen Drehung des Buchsenteils und des Kragens gestaltet sind, wobei die genannten Kerben (10) des Kragens durch Querverformung des Abschnitts (7b) des Kragens, der die Kerben trägt, seitlich von den Kerben (6) des Buchsenteils entfernt werden können.

2. Verbinder nach Anspruch 1, dessen genannter Kragen zwei Reihen von Kerben (10), die einander diametral gegenüberliegen, und zwei Bereiche (11) in 90° zu den Kerben umfasst, die dazu geeignet sind, zur Querverformung des Kragens radial geschoben zu werden, um die zwei Reihen von Kerben voneinander abzurücken, um die Trennung des Kragens und des Buchsenteils des Verbinders zu ermöglichen.

3. Verbinder nach Anspruch 1 oder 2, deren Kerben (6, 10) des Kragens und des Buchsenteils eine leichte Steigung (6a, 10a) zur Erleichterung und Automatisierung der Verriegelung durch axialen Schub und eine starke Steigung (6b, lOb) zur Verhinderung der Entriegelung aufweisen, wobei die Verriegelung durch den Kontakt der starken Steigungen der Kerben des Kragens mit den leichten Steigungen der Kerben des Buchsenteils sichergestellt wird.

4. Verbinder nach einem der Ansprüche 1 bis 3, in dem die Kerben (6) des Buchsenteils Rillen oder Abschnitte von kreisförmigen Rillen sind.

5. Verbinder nach einem der Ansprüche 1 bis 4, dessen Steckerteil (1) an seinem Ende zum Einpassen (1a) zum Aufnehmen eines Luer-Lock-Steckers (LM) mittels Pressverbindung entworfen ist.

6. Verbinder nach einem der Ansprüche 1 bis 4, dessen Buchsenteil (4) an ihrem Ende zum Einpassen (4a) innen mit einem Luer-Lock-Stecker (LM) entworfen ist, der dazu geeignet ist, in eine Luer-Lock-Buchse (LF) eingepresst zu werden.

7. Verbinder nach einem der Ansprüche 1 bis 4, dessen eines und/oder anderes Ende zum Einpassen zur Verbindung mit einem zylindrischen Rohr entworfen ist bzw. sind.

8. Anwendung eines Verbinders nach einem der Ansprüche 1 bis 7 zum Anschluss einer Spritze oder eines Nadel- oder Katheteransatzstücks.

9. Anwendung eines Verbinders nach einem der Ansprüche 1 bis 7 zum Anschluss eines Rohrs.
